Europäisches Patentamt

European Patent Office

Office européen des brevets

(11) Publication number: **0 054 067**
**B1**

(12) **EUROPEAN PATENT SPECIFICATION**

(45) Date of publication of patent specification: **18.09.85**

(21) Application number: **81901963.9**

(22) Date of filing: **18.06.81**

(86) International application number:
**PCT/US81/00823**

(87) International publication number:
**WO 81/03666 24.12.81 Gazette 81/30**

(51) Int. Cl.⁴: **C 12 P 7/26,** C 12 P 19/02,
C 12 N 11/00

(54) **PROCESS FOR MAKING FRUCTOSE.**

(30) Priority: **18.06.80 US 160762**

(43) Date of publication of application:
**23.06.82 Bulletin 82/25**

(45) Publication of the grant of the patent:
**18.09.85 Bulletin 85/38**

(84) Designated Contracting States:
**DE FR GB NL SE**

(56) References cited:
**EP-A-0 042 221**
**CS-A- 175 897**
**US-A-3 651 221**
**US-A-4 246 347**
**US-A-4 247 641**

(73) Proprietor: **STANDARD BRANDS
INCORPORATED
15 River Road
Wilton, CT 06897 (US)**

(72) Inventor: **MASELLI, John A.
43 Old Highway
Wilton, CT 06897 (US)**
Inventor: **HORWATH, Robert O.
223 Bayberry Lane
Westport, CT 06880 (US)**

(74) Representative: **Lehn, Werner, Dipl.-Ing. et al
Hoffmann, Eitle & Partner Patentanwälte
Arabellastrasse 4 (Sternhaus)
D-8000 München 81 (DE)**

Courier Press, Leamington Spa, England.

# Description

This invention is concerned with a new and useful process for the production of glucosone and more particularly for the production of glucosone from which food-grade fructose can be obtained.

Commercial methods for the production of fructose, a commercially important sweetener, primarily involve a two-step process, the first, hydrolysis of a polysaccharide such as starch to produce glucose and the second, isomerization of the so-produced glucose to form fructose. The latter step, as is well-known, produces a mixture of glucose and fructose from which it is difficult to separate the desired product, fructose. The commercial separation method involves the use of crystallization techniques which are costly and time-consuming. More detailed description of the various methods of isomerizing glucose can be found in the literature, e.g., U.S. Patent 3,788,945 and 3,616,221.

Glucose can also be converted to fructose by the action of an enzyme, designated glucose-2-oxidase, to form glucosone (D-arabino-2-hexosulose) which in turn can be reduced to fructose with zinc and acetic acid [Folia Microbiol. 23, 292—298 (1978) and Czechoslovakian Patent No. 175897 to Volc et al.].

The reaction of glucose-2-oxidase with glucose to produce glucosone also yields hydrogen peroxide in equimolar amount. The use of the so-produced hydrogen peroxide in the conversion of alkenes to corresponding halohydrins and epoxides has been proposed in European Patent Application 7176. In the published application, the *in situ* formation of hydrogen peroxide is proposed by inclusion of glucose-2-oxidase and glucose in the reaction mixture which includes a halogenating enzyme and a source of inorganic halide into which the selected alkene is to be introduced. The disclosure of the European patent application further indicates that the glucosone product of the enzymatic oxidation of glucose can be converted to fructose by simple chemical hydrogenation.

However, fructose produced by the said process can be contaminated with significant amounts of by-products from both the enzymatic conversion of glucose and the alkene conversion reaction. In particular, the latter reaction produces halohydrins and alkylene oxides, e.g. ethylene oxide, which are highly toxic materials even at levels in the region of parts per million. Thus, fructose produced by such a process will require careful and costly purification to attain food grade purity. Further, the potential for contamination of fructose by virtue of secondary reactions during the initial processing stage is quite high due to the highly reactive products, halohydrins and alkyleneoxides, and substantial purification procedures are required to assure the high level purity required for food grade fructose.

This invention provides a method for the production of glucosone and is characterized in that glucose is enzymatically oxidized with glucose-2-oxidase in the presence of an enzyme which reduces hydrogen peroxide dissolved in said oxidation mixture (first embodiment) or in the presence of a platinum metal to decompose hydrogen peroxide which is concomitantly produced (second embodiment).

In the first embodiment of the invention it is particularly preferred that the hydrogen peroxide-reacting enzyme which reduces hydrogen peroxide is suspended in the enzymatic oxidation medium in immobilized form. Enzyme immobilization is familiar to those skilled in the art. The processes for immobilization generally involve reaction of the enzyme, usually in aqueous solution with either organic or inorganic supports. The most common of these supports are organic polymers, such as acrylic acid and methacrylic acid polymers and copolymers, as well as the corresponding esters and amides, e.g. poly acrylamide and polybutylmethacrylate, styrene polymers and copolymers, cellulose, agarose, and polypeptides. Inorganic supports include alumina, silica, titania, porous glass beads, as well as gels of alumina and titania.

As a further preferred form of this first embodiment, the oxidizing enzyme, glucose-2-oxidase, is also provided in an insoluble form, e.g. as immobilized enzyme, immobilized microorganism containing the enzyme, or the microorganism containing the enzyme. With both enzyme systems being readily removable by simple separation techniques, filtration or centrifugation, the resulting reaction mixture which comprises principally glucosone is substantially free of undesired contaminants and can be processed to fructose of food grade quality.

In the second embodiment of the invention platinum can be provided in any form which can be distributed throughout the enzymatic oxidation medium. In particular, a metal grid coated with platinum is especially effective in that it provides a high surface area of platinum. A plurality of said grids can be arranged in the oxidative medium to permit more efficient decomposition of the hydrogen peroxide produced during the enzymatic oxidation of glucose.

The use of the present process results in considerable advantage particularly in the further processing of glucosone to fructose. The removal of hydrogen peroxide by reaction from the reaction medium of course affects the rate of the enzymatic oxidation of glucose so that the reaction tends to be more complete and the reaction times can be shorter than normally required. Further, the reaction medium is essentially free of contaminants.

The glucosone solution produced in the reaction mixture can be used as such in the hydrogeneation step or can be concentrated or otherwise processed as desired. The glucosone solution is substantially free of contaminants other than some unreacted glucose, or glucose dimer or trimer, and whatever contaminants that may have been introduced in the original glucose charge. Usually, the glucose charge will be a hydrolysate of a natural product containing glucose units, most commonly starch, which will contain soluble contaminants such as other carbohydrates, e.g. maltose, formed in the starch hydrolysis.

Accordingly, the reduction of the reaction product will provide a product, fructose, which will be comparatively free of contaminants that effect food grade status for the product, the contaminants being derived only from the glucose natural sources, e.g. starches such as corn starch.

The glucose-2-oxidase enzyme can be provided in the form of the enzyme solution in water, immobilized enzyme or immobilized cells or mycelium or the free cells or mycelium. Most commonly since the enzyme is intracellular, the cells or mycelium of the selected microorganism are used by merely suspending them in the reaction solution. Promoters and protectors for the enzyme can also be present. For example, as described in the aforesaid Folia Microbiol. 23, 292—298 (1978), the presence of fluoride ion promotes the enzymatic oxidation of glucose with O. mucida. Protectors for enzymes can also be used, e.g. Co, Mn and Mg salts.

The enzymatic oxidation reaction is carried out until substantially complete as can be determined by monitoring the mixture using aliquots to test for glucose content, or by colorimetric determination of glucosone or by determination of hydrogen peroxide. Usually, reaction periods of about 24—48 hours are sufficient, depending on enzyme potency or activity.

A wide variety of microorganisms can be used to produce the glucose-2-oxidase employed in the present process. For example, the following organisms are described in the literature for this purpose:

    I Aspergillus parasiticus [Biochem. J. 31, 1033 (1937)]
    II Iridophycus flaccidum [Science 124, 171 (1956)]
    III Oudemansiella mucida [Folia Microbiol. 13, 334 (1968) ibid. 23, 292—298 (1978)]
    IV Gluconobacter roseus [J. Gen Appl. Microbiol. 1, 152 (1955)]
    V Polyporous obtusus [Biochem. Biophys. Acta 167, 501 (1968)]
    VI Corticium caeruleum [Phytochemistry 1977 Vol. 16, p 1895—7]

The temperature for the enzymatic oxidation reaction is not critical. The reaction can be conducted at room temperature, or even somewhat higher than room temperature where the enzyme system employed is of reasonable heat stability. In particular, it is preferable to operate at 50°C and above, with heat stable enzyme systems in which range bacterial infection of the reaction mixture is minimized. Alternatively, the enzymatic reaction mixture can contain antibacterial agents to preclude extensive bacterial growth.

The reaction medium of course should contain no significant amounts of a reducing agent for hydrogen peroxide other than the aforesaid enzymes or platinum so that the beneficial results of the present process can be realised. Thus, the system should be substantially free of reducing agents for $H_2O_2$, i.e. a non-reducing system, except for the presence of the specifically added enzymes or platinum.

The source of the reducing enzyme for the removal of hydrogen peroxide is any source of peroxidase or catalase, preferably a food source which is compatible with the food grade status desired for the final product fructose. Thus, yeast, milk, eggs, horseradish and similar such sources are usually preferred, whether the enzyme is employed as such or in the immobilized state.

The reduction of glucosone to fructose is accomplished by known procedures including chemical reduction as with zinc and acetic acid as well as catalytic hydrogenation, with the usual metal catalysts. Of these, the preferred metal catalyst is Raney Ni since its use is compatible with the desired food grade of fructose, i.e. no residues or contaminants are left by this catalyst.

In the usual procedure employed, the glucosone is hydrogenated at elevated pressure and temperature over the selected metal catalyst until the desired degree of hydrogenation has been achieved. Pressures can range from 100 to 700 bar and even higher while the temperature can range up to about 200°C. Preferred is 100° to 150°C and a pressure of about 500 bar.

The following example further illustrates the invention.

### Example 1

Mycelium of O. mucida are grown in accordance with Example 1 of Czechoslovakian patent 175897 and the equivalent of 15 g (dry weight) of the mycelium is suspended in 3 L of 2.5% glucose solution of 0.05 M NaF in a 10 L reactor. Catalase enzyme immobilized on DEAE-cellulose (Cellex-D®, manufactured by Bio-Rad Laboratories) is suspended in glucose solution.

The suspension is mixed at 25°C and aerated with oxygen. After 24 hours the mycelium and immobilized enzyme is then separated from the solution in the first zone and the resulting clear solution is then hydrogenated over Raney Ni at 500 atmospheres hydrogen gas and 100°C. The aqueous mixture is filtered clear of the catalyst, decolorized with carbon, dionized with ion-exchange (anionic and cationic), and concentrated to a fructose syrup at reduced pressure. Alternatively, the aqueous mixture is concentrated and fructose allowed to crystallize.

The fructose obtained as either syrup or crystalline product is of food grade quality.

Essentially the same results are obtained when O. mucida is replaced with the following organisms:

    Polyporus obtusus
    Radulum casearium
    Lenzites Trabea
    Irpex flanus
    Polyporus versicolor
    Pellicularia filamentosa Armillaria mellea
Schizophyleum commune
Corticium caeruleum

Example 2

The procedure of Example 1 is repeated with several metal grids coated with platinum replacing the immobilized catalase enzyme with substantially the same results.

Example 3

The procedure of Example 1 is repeated using an aqueous solution of the catalase enzyme in lieu of the immobilized enzyme with essentially the same results.

**Claims**

1. A method of producing glucosone which comprises enzymatically oxidizing glucose with glucose-2-oxidase in the presence of an enzyme which reduces hydrogen peroxide dissolved in said oxidation mixture.

2. The method according to Claim 1 wherein the reducing enzyme is in immobilized form suspended in the reaction mixture.

3. The method according to Claim 1 or 2 wherein said reducing enzyme is a catalase or a peroxidase.

4. The method according to any of Claims 1—3 wherein said reducing enzyme is a catalase.

5. The method according to any of Claims 1—3 wherein said reducing enzyme is a peroxidase.

6. The method according to any of Claims 1—5 wherein said glucose-2-oxidase is present in immobilized form suspended in the reaction mixture.

7. The method according to any of Claims 1—5 wherein said glucose-2-oxidase is present in the microorganism from which derived.

8. A method of producing glucosone which comprises enzymatically oxidizing glucose with glucose-2-oxidase in the presence of platinum metal to decompose hydrogen peroxide which is concomitantly produced.

9. The method according to Claim 8 wherein said platinum metal is provided in form of at least one grid suspended in the oxidation medium.

10. The method according to Claim 8 wherein a plurality of said grids is employed.

11. The method according to any of Claims 1—10 including the further step of reducing the so-produced glucosone to obtain fructose.

12. The method according to Claim 12 wherein the reduction is effected by catalytic hydrogenation.

13. The method according to Claim 12 wherein the catalyst is Raney Ni.

14. The method according to any of Claims 11—13 wherein the filtered reaction mixture obtained from the enzymatic oxidation process is employed as the substrate for reduction to fructose.

**Revendications**

1. Procédé de préparation de glucosone qui consiste à oxyder enzymatiquement le glucose par le glucose-2-oxydase en présence d'une enzyme qui réduit le peroxyde d'hydrogène dissous dans le mélange d'oxydation.

2. Procédé selon la revendication 1, dans lequel l'enzyme réductrice est sous forme immobilisée en suspension dans le mélange réactionnel.

3. Procédé selon l'une des revendications 1 et 2, dans lequel l'enzyme réductrice est une catalase ou une peroxydase.

4. Procédé selon l'une des revendications 1 à 3, dans lequel l'enzyme réductrice est une catalase.

5. Procédé selon l'une des revendications 1 à 3, dans lequel l'enzyme réductrice est une peroxydase.

6. Procédé selon l'une des revendications 1 à 5, dans lequel le glucose-2-oxydase est présent sous forme immobilisée en suspension dans le mélange réactionnel.

7. Procédé selon l'une des revendications 1 à 5, dans lequel le glucose-2-oxydase est présent dans le microorganisme dont il est dérivé.

8. Procédé de préparation de glucosone qui consiste à oxyder enzymatiquement le glucose par le glucose-2-oxydase en présence d'un métal du groupe du platine pour décomposer le peroxyde d'hydrogène qui se forme de façon concomitante.

9. Procédé selon la revendication 8, dans lequel le métal du groupe du platine est prévu sous la forme d'au moins une grille suspendue dans le milieu d'oxydation.

10. Procédé selon la revendication 8, dans lequel on utilise plusieurs grilles.

11. Procédé selon l'une des revendications 1 à 10, comprenant l'étape supplémentaire de réduction de la glucosone ainsi préparée pour l'obtention de fructose.

12. Procédé selon la revendication 11, dans lequel on effectue la réduction par hydrogénation catalytique.

13. Procédé selon la revendication 12, dans lequel le catalyseur est le nickel Raney.

14. Procédé selon l'une des revendications 11 à 13, dans lequel on utilise le mélange réactionnel fitré tiré du processus d'oxydation enzymatique comme substrat pour la réduction en fructose.

**Patentansprüche**

1. Verfahren zur Herstellung von Glucoson, gekennzeichnet durch enzymatische Oxidation von Glucose mit Glucose-2-oxidase in Gegenwart eines Enzyms, welches das in dem genannten Oxidationsgemisch gelöste Wasserstoffperoxid reduziert.

2. Verfahren nach Anspruch 1, dadurch gekennzeichnet, dass das reduzierende Enzym in immobilisierter Form suspendiert in dem Reaktionsgemisch vorliegt.

3. Verfahren nach Anspruch 1 oder 2, dadurch gekennzeichnet, dass das reduzierende Enzym eine Katalase oder eine Peroxidase darstellt.

4. Verfahren nach einem oder mehreren der Ansprüche 1 bis 3, dadurch gekennzeichnet, dass das reduzierende Enzym eine Katalase darstellt.

5. Verfahren nach einem oder mehreren der Ansprüche 1 bis 3, dadurch gekennzeichnet, dass das reduzierende Enzym eine Peroxidase darstellt.

6. Verfahren nach einem oder mehreren der Ansprüche 1 bis 5, dadurch gekennzeichnet, dass die genannte Glucose-2-oxidase in immobilisierter Form suspendiert in dem Reaktionsgemisch vorliegt.

7. Verfahren nach einem oder mehreren der Ansprüche 1 bis 5, dadurch gekennzeichnet, dass die genannte Glucose-2-oxidase in dem Mikroorganismus vorliegt, der dieselbe produziert.

8. Verfahren zur Herstellung von Glucoson, gekennzeichnet durch enzymatische Oxidation von Glucose mit Glucose-2-oxidase in Gegenwart von Platinmetall zur Zersetzung des gleichzeitig gebildeten Wasserstoffperoxids.

9. Verfahren nach Anspruch 9, dadurch gekennzeichnet, dass das genannte Platinmetall in Form mindestens eines Netzes, welches in dem Oxidationsmedium suspendiert ist, vorliegt.

10. Verfahren nach Anspruch 8, dadurch gekennzeichnet, dass mehrere der genannten Netze bzw. Gitter verwendet werden.

11. Verfahren nach einem oder mehreren der Ansprüche 1 bis 10, dadurch gekennzeichnet, dass es als weiteren Schritt die Reduzierung des auf diese Weise hergestellten Glucosons unter Erhalt von Fructose umfasst.

12. Verfahren nach Anspruch 11, dadurch gekennzeichnet, dass die Reduktion durch katalytische Hydrierung durchgeführt wird.

13. Verfahren nach Anspruch 12, dadurch gekennzeichnet, dass der Katalysator Raney-Ni darstellt.

14. Verfahren nach einem oder mehreren der Ansprüche 11 bis 13, dadurch gekennzeichnet, dass das filtrierte Reaktionsgemisch, das bei dem enzymatischen Oxidationsverfahren erhalten wird, als Substrat zur Reduktion zu Fructose dient.